# EUROPEAN PATENT SPECIFICATION

(11) **EP 0 505 389 B1**
(45) Date of publication and mention of the grant of the patent: **14.05.1997**
(21) Application number: 90917621.6
(22) Date of filing: 09.11.1990
(51) Int. Cl.: A01N 57/00, A61K 31/675

(54) **ANTIPARASITIC COMPOSITION FOR ANIMAL USE**
ANTIPARASITÄRE ZUSAMMENSETZUNG ZUR VERWENDUNG BEI TIEREN
COMPOSITION ANTIPARASITAIRE POUR ANIMAUX

(30) Priority: 19.12.1989 JP 330224/89; 26.12.1989 JP 338973/89; 27.04.1990 JP 113147/90; 13.07.1990 JP 18613/90
(43) Date of publication of application: 30.09.1992
(73) Proprietor: SMITHKLINE BEECHAM CORPORATION, Philadelphia Pennsylvania 19101 (US); Takeda Chemical Industries, Ltd., Chuo-ku, Osaka (JP)
(72) Inventor: PARISH, Roger, King of Prussia, PA 19406 (US); CHAPIN, Frederic, W., West Chester, PA 19382 (US); KONO, Yoshiaki, Shizuoka (JP); TSUKUI, Makoto 3-2-17, Kaname-cho 3-chome, Tokyo (JP)
(74) Representative: Moore, James William
(86) International application number: US9006595
(87) International publication number: WO9108669

(56) References cited:
- EP-A- 0 189 377
- CA-A- 1 177 081
- US-A- 4 140 774
- US-A- 4 531 005
- US-A- 4 621 144
- US-A- 4 781 920

## Description

The present invention relates generally to an antiparasitic composition and its use for treating animals infected with internal and external parasites, such as helminths, nematodes, ticks and others. More specifically the invention relates to a composition containing albendazole and the organophosphate, pyraclofos, and its use in treating infections due to benzimidazole-resistant parasitic infections.

### Background of the Invention

Parasitic infections of animals, particularly domesticated animals such as cattle, horses, sheep and swine, poultry or pet animals, have long been a problem for owners and breeders alike. The major groups of animal parasites are found among the Protista, the helminths (round worms and flat worms) and the arthropods. Helminthiasis is an infection of the host animal caused by parasites called helminths. Helminthiasis is a serious epidemic in domesticated animals such as swine, sheep, horses, cattle, goats, dogs and cats and in poultry. Among helminths, the group of parasites known as nematodes or round worms cause universal and serious infections in various species of animals. The genera of parasites which are most commonly found to affect the above-mentioned animals are Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascardia, Oxyuris, Ancylostoma, Uncinaria, Toxascaris and Parascaris. Whereas some nematodes, such as Cooperia and Oesophagostomum, primarily attack the intestinal canal, others such as Haemonchus and Ostertagia are present most abundantly in the stomach. Dictyocaulus and others are found in the lungs. Still other parasites may inhabit other tissues and organs of the body, such as the heart, blood vessels, and subcutaneous and lymph tissues. The parasitic infection, known as helminthiasis, causes anemia, malnutrition, body weight loss and severe damage to the intestinal wall and other tissues and organs and, if left untreated, may lead to death of the host animal.

Other parasitic infections with various species of arthropods, e.g., ticks and mites, fleas, lice, flies, mosquitoes, while the cause of less severe health problems for the animals, such as itching, uncleanliness and discomfort, are also carriers of internal parasites such as helminths, for example, trematodes, cestodes, and nematodes which cause fatal diseases in the animals.

A variety of anthelmintic compositions presently exist to treat internal parasites, and other compositions exist to treat the external parasites. Among such compositions are various compounds called benzimidazoles and benzimidazole carbamates. See, for example, U. S. Patent Nos. 3,956,499; 3,574,845 and 3,915,986. Among such compounds are albendazole [methyl 5-(n-propylthio)-1H-benzimidazol-2-ylcarbamate], fenbendazole [methyl 5-(phenylthio)-1H-benzimidazol-2-ylcarbamate], oxibendazole [methyl 5-(n-propoxy)-1H-benzimidazol-2-ylcarbamate], oxfendazole [methyl 5-(phenylsulfinyl)-1H-benzimidazol-2-ylcarbamate], parbendazole, cambendazole, mebendazole, flubendazole, ricobendazole, luxabendazole and others. These benzimidazoles are commercially available. For example, albendazole is presently marketed for use as a cattle dewormer under the brand name "Valbazen" [SmithKline Beecham Animal Health Laboratories].

However, various species of helminths have developed resistance to several of these benzimidazoles. Particularly albendazole alone is becoming an ineffective control agent against ever increasing populations of benzimidazole-resistant nematodes. Therefore several benzimidazoles have been formulated in combination with various organophosphate compounds for similar use. For example, US Patent 4,436,737 provides anthelmintic compositions containing benzimidazoles and the organophosphate, profenofos. Profenofos has the formula C₁₁H₁₅ClBrO₃PS. The combination product (albendazole and profenofos) was employed against benzimidazole-resistant helminths, such as Haemonchus contortus and Trichostrongylus colubriformis.

South African patent 6602811 provides an anthelmintic composition containing an organophosphate and oxibendazole or thiabendazole.

Japanese patent 59033204 discloses a fungicide containing an organophosphate and thiabendazole.

European Patent 181525 discloses an anthelmintic combination of tetramisole with several organophosphates, including chlorpyrifos.

U. S. Patent 3,992,533 refers to thiophosphoric acid phenyl esters as a broad insecticidal agent against plant pathogenic pests.

UK Patent Application 2,094,625 refers to an anthelmintic composition containing alkyl S-substituted benzimidazolyl carbamate and O-ethyl-O-(4-bromo-2-chlorophenyl)-3-propyl phosphorothioate.

U.S. Patent 3,244,586 refers to the organophosphate chlorpyrifos, a compound of formula C₉H₁₁Cl₃NO₃PS.

U. S. Patent 4,531,005 refers to a number of organophosphate compounds, including pyraclofos, useful as insecticides for plant.

U. S. Patent 4,531,005 refers to 4-pyrazolyl phosphorous acid esters useful as intermediates for the production of 4-pyrazolyl phosphorothioates. These latter compounds are noted as agricultural insecticides and acaricides.

There remains a need in the art for effective antiparasitic compositions for use in treating or preventing parasitic infections, particularly those parasites which are naturally resistant or which have developed resistance to prior art compositions.

### Summary of the Invention

As one aspect, the invention provides an antiparasitic composition for the prevention, treatment control and/or removal of internal and external parasites from animals which can be administered to animals in effective dosages which do not cause adverse toxic effects in animals. In one embodiment, the composition comprises the combination of pyraclofos or a pyraclofos-related compound and a benzimidazole carbamate or pro-drug derivative thereof as the active ingredients. further embodiment, the benzimidazole of the composition is albendazole.

As a further aspect, the invention provides a formulation enabling internal administration of the antiparasitic compositions of this invention for the treatment of endoparasitic infection.

Yet other aspects of this invention include appropriate dosage units of the antiparasitic formulations of this invention.

Other aspects and advantages of the present invention are described further in the following detailed description of preferred embodiments thereof.

### Detailed Description of the Invention

The present invention provides an anti-parasitic, particularly anthelmintic, composition for use in the prevention, treatment, control and/or removal of various parasitic infections in animals, caused by both internal parasites, such as helminths and external parasites, such as arthropods. Among the utilities of the present composition is the treatment of parasitic infestations caused by parasites which are resistant to other antiparasitic compositions, particularly the popular anthelmintic compound, albendazole.

The inventors have discovered that the organophosphate compound, pyraclofos, a known plant insecticide, can be effectively employed as an active ingredient in compositions for the removal or control of endoparasites in animals. It has also been surprisingly discovered that pyraclofos can be internally, preferably orally, administered to control animal ectoparasites, as well. Pyraclofos can be formulated for safe internal administration to an animal, in combination with known antiparasitic compounds, for example, benzimidazoles or benzimidazole carbamates, such as albendazole.

The chemical name for pyraclofos is O-[1-(4-chlorophenyl)-1H-pyrazol-4-yl]-O-ethyl-S-propyl phosphorothioate. The structural formula of this compound is 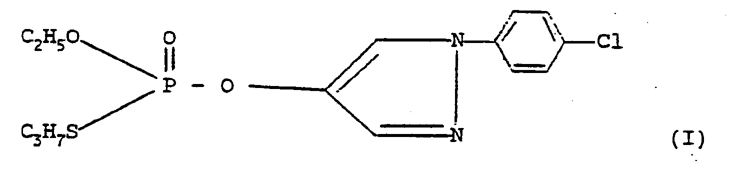 This compound and methods for producing it are described in detail in U. S. Patent 4,531,005, particularly Example 18 therein. This patent is incorporated herein by reference for the purpose of providing information on this compound. Pyraclofos can be obtained under the trade name "Boltage" [Takeda Industries, Japan].

The inventors have discovered that in combination with a selected benzimidazole, an anthelmintic composition is formed which is characterized by superior efficacy and non-toxicity in animals.

This composition including pyraclofos and a benzimidazole is particularly efficacious against parasites which are substantially resistant to benzimidazoles in general, or albendazole specifically, namely the nematodes, Haemonchus contortus, Ostertagia circumcincta, and Trichostrongylus colubriformis.

While pyraclofos is specifically disclosed in the compositions and formulations exemplified herein, it may be possible to substitute for pyraclofos in the above compositions, certain compounds hereafter referred to as "pyraclofos-related compounds". Pyraclofos-related compounds include pyrazolyl phosphoric esters having the general formula 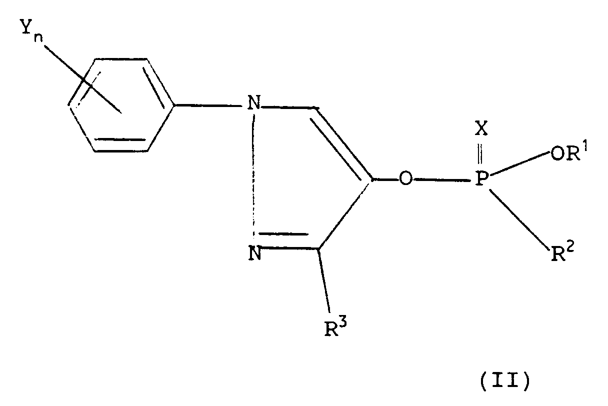 wherein R¹ is a lower alkyl group; R² is a lower alkoxy group or a lower alkylthio group; R³ is a hydrogen atom or a lower alkoxy carbonyl group; X is an oxygen atom or a sulfur atom; Y is a lower alkyl group, a lower alkoxy group, a lower alkylthio group, a halogen atom, a nitro group or a trifluoromethyl group; and n is an integer of 0, 1, 2 or 3.

Referring to general formula (II), R¹ for a lower alkyl group means a straight-chain or branched alkyl group of 1 to 4 carbon atoms, such as methyl, ethyl, n-propyl, isopropyl, n-butyl, isobutyl, sec-butyl and so on. R² for a lower alkoxy group means an alkoxy group of 1 to 4 carbon atoms, such as methoxy, ethoxy, n-propoxy, isopropoxy, n-butoxy, isotuboxy, sec-butoxy and so on. R² for a lower alkylthio group means an alkylthio group containing 1 to 4 carbon atoms, such as methylthio, ethylthio, n-propylthio, isopropylthio, n-butylthio, isobutylthio, sec-butylthio and so on. R³ for a lower alkoxycarbonyl group means an alkoxycarbonyl group of 2 to 5 carbon atoms, such as methoxycarbonyl, ethoxycarbonyl and so on. Of the groups denoted by Y, the lower alkyl group, lower alkoxy group and lower alkylthio group means the same groups as defined for R¹ and R², respectively, although t-butyl may also be used as the lower alkyl group. The halogen atom, also represented by Y, includes fluorine, chlorine, bromine and iodine. The symbol n denotes the number of substituents represented by Y and where two or more such substituents are present, they may be the same or different. Furthermore, where the group Y is a lower alkoxy group and n is equal to 2, the two alkoxy groups may, taken together, represent an alkylidenedioxy group, such as methylenedioxy, propylidenedioxy, etc., which also falls within the scope of the invention.

Among the pyraclofos-related compounds that may be useful in the invention as substitutions for pyraclofos, the compounds having substituents of Formula II as listed in Table 1 below, may be of particular importance. Furthermore, the compounds of formula (II) wherein R¹ is ethyl and R² is n-propylthio are particularly desirable.

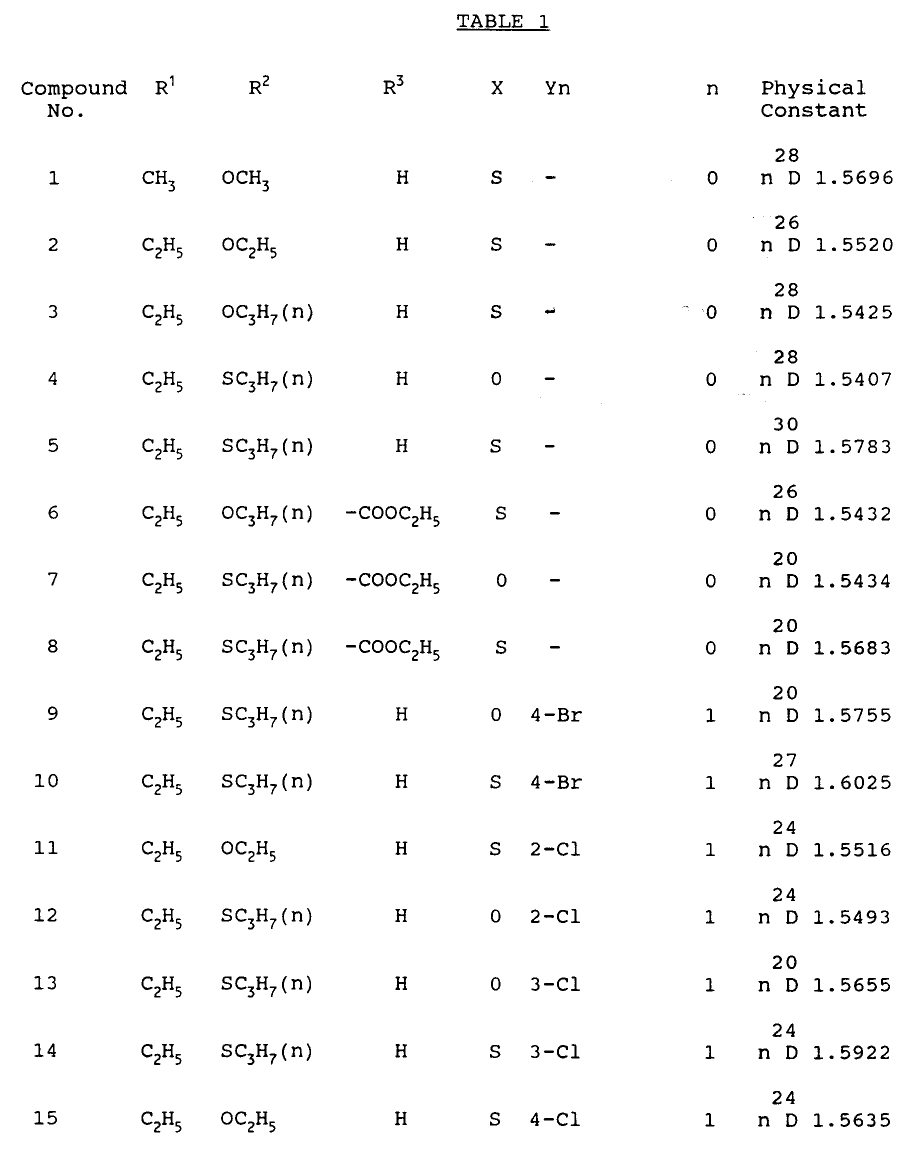

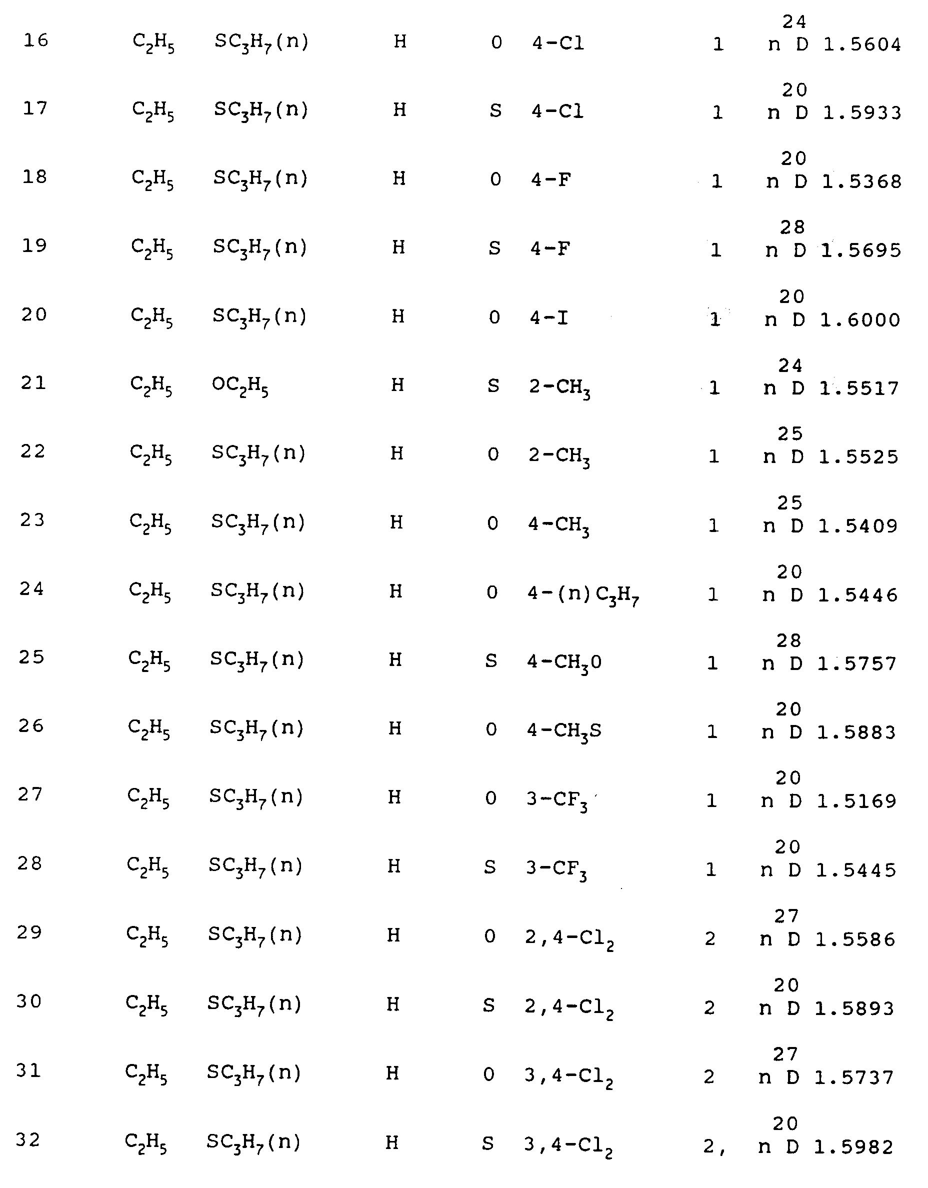

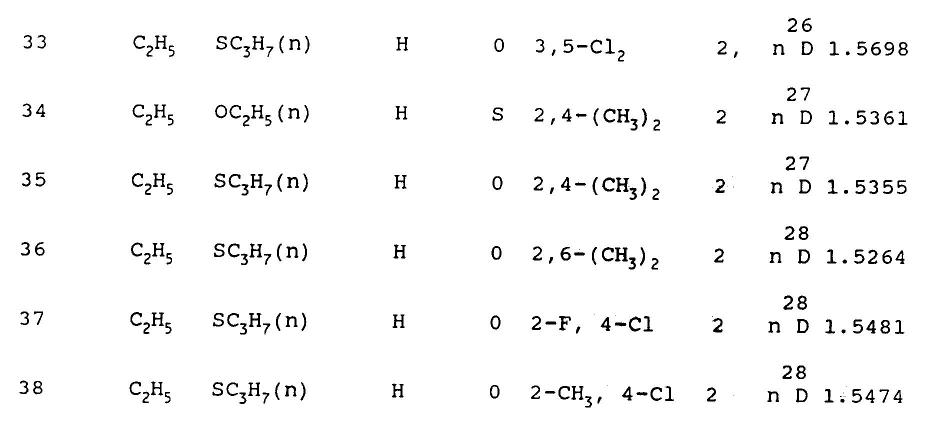

The presently most preferred benzimidazole for the combination compositions with pyraclofos or its related compounds is albendazole [methyl 5-(n-propylthio)-1H-benzimidazol-2-ylcarbamate], as specifically disclosed in the compositions and formulations exemplified herein. However, it may be possible to substitute other known and commercially available benzimidizoles or prodrugs which metabolize in the animal into a suitable benzimidazole for albendazole in these compositions. Among such other benzimidizoles are included the following:
Fenbendazole [methyl 5-(phenylthio)-1H-benzimidazol-2-ylcarbamate]
Oxibendazole [methyl 5-(n-propoxy)-1H-benzimidazol-2-ylcarbamate]
Oxfendazole [methyl 5-(phenylsulfinyl)-1H-benzimidazol-2-ylcarbamate]
Parbendazole [methyl 5-(butyl)-1H-benzimidazol-2-ylcarbamate]
Cambendazole [isopropyl 2-(4-thiazolyl)-5-benzimidazolecarbamate]
Mebendazole [methyl 5-benzoyl-2-benzimidizolecarbamate] and
Flubendazole [methyl 5-(4-fluorobensolyl)-1H-benzimidazol-2-ylcarbamate]. Ricobendazole, and Luxabendazole are also known benzimidazoles. These benzimidazoles may be prepared by one of skill in the art with resort to known techniques such as those described in the art cited above.

Prodrugs which metabolise in vivo into selected benzimidazoles may also be employed to replace albendazole in the compositions of this invention. Among the known prodrugs are thiophanate [1,2-phenylenebis (iminocarbonothioyl)biscarbamic acid diethyl ester ]. See, for example, U. S. Patent Nos. 3,745,187 and 3,769,308. Another prodrug which may be useful is febantel, i.e., [2-[(methoxyacetyl)amino]-4-(phenylthio)-phenyl] carbonimidoyl] biscarbamic acid dimethyl ester. Also useful may be netobimin. These known compounds may also be prepared by known and conventional methods by one of skill in the art.

The antiparasitic compositions of the present invention containing pyraclofos are effective in substantially reducing the number and/or totally eliminating the internal and external parasites. These compositions are also effective upon topical application of warding off both internal and external parasites from animals. Additionally the compositions of this invention have the characteristic of substantial non-toxicity to animals when orally administered and ingested by animals at effective dosages.

While the compositions of the invention are particularly useful for the treatment, prevention or management of helminthiasis, caused particularly by benzimidazole-resistant helminths, such compositions may also be useful, also, for the treatment of diseases caused by other parasites.

Depending upon the particular use for which a composition of this invention is directed, the antiparasitic compositions of the invention can be administered to animals in accordance with the procedures known to the profession in the field of veterinary medicine. For example, in the treatment of ectoparasites and endoparasites of animals, e.g., ticks which feed through the surface of the animal's skin and helminths which parasitize internally, the compositions of this invention may be administered to such animals internally.

While internal administration is preferably by the oral route, other modes of internal administration, including without limitation, direct administration into the rumen, intraperitoneal, intramuscular, intratracheal, intravenous or subcutaneous administration, may also be employed.

Compositions of this invention may be prepared by dispersing the pyraclofos or related compounds, preferably in combination with albendazole, uniformly in an inert vehicle or diluent. The term "inert vehicle" means any pharmaceutical vehicle that does not react with the pyraclofos or albendazole and can be safely administered to animals.

These compositions may be formulated into various oral dosage forms such as capsules, pills, tablets, granules, solutions, oral pastes and other standard oral formulations. For oral administration, the compositions of this invention can also be evenly admixed into the animal ration, dissolved or suspended in drinking water or admixed in one of the dosage forms described above. In certain cases, oral dosage forms preparations may be given to animals independently of feeding. The vehicle for dietary administration is preferably a component of the ration or a substance which can be a component of the ration. The vehicle or diluent suited to such compositions includes, among others, malt clearings, corn meal, brewers' grains, crushed oyster shell, wheat bran, soluble molasses, corncob meal, soybean meal and crushed limestone. The active compound is intimately dispersed throughout such vehicle, for example by grinding, stirring, milling or rotational agitation.

For internal administration by oral and other internal delivery routes, the compositions may be prepared by dissolving or dispersing pyraclofos and/or albendazole in a liquid vehicle. Among desirable liquid vehicles are vegetable oils, for example, peanut oil, cottonseed oil, corn oil, water, and physiological saline solution. Presently most desirable for the combination formulation containing pyraclofos and albendazole is corn oil.

Preparations of the above-mentioned dosage forms can be manufactured by conventional pharmaceutical procedures, by adding to the active ingredient(s) diluents, disintegrating agents, antifoams, lubricants, dispersants, surfactants, binders, and emulsifiers. These optional ingredients may be selected from starch, lactose, talc, magnesium stearate, colloidal silicon dioxide, microcrystalline cellulose, dicalcium phosphate, bentonite, calcium sulfate demihydrate, hydroxypropylmethylcellulose, corn starch, sodium starch glycolate, sodium laurylsulfate, Polysorbate 85 (Sanyo Chemical), phenyl sulfonate CALX (ICI), Synperonic NP13 (ICI), Synperonic PE L44 (ICI), benzyl alcohol, propylene glycol, vegetable resin (wood resin) and so on. Emulsifiers, such as solketal, glycerol formal, benzyl alcohol, carboxymethylcellulose, Sorpol 1200 (Toho Chemical), Polysorbate 80 (Sanyo Chemical), cetyl alcohol and Brij 58 (ICI), may be employed. The latter two emulsifiers are desirable for the combination compositions. Further an optional stiffening agent, e.g., Cabosil (Cabot) may also be added.

The presently most desirable formulation of the combination of pyraclofos and albendazole employs corn or other vegetable oil as the liquid vehicle with optimal concentrations of 1% Brij 58, 1% cetyl alcohol and 1% colloidal silica. This formula is preferred for intraruminal administration, but may also be employed orally. This formula, when the albendazole and pyraclofos are homogenized therein, readily suspends, remains flowable at low temperatures, of about 5°C. Optional components of this formulations included a flavoring agent and a dye.

Dosages of the active ingredients, pyraclofos alone or pyraclofos and albendazole in combination can vary widely in the total weight of the formulation. The percentage content of the active ingredients may also be varied according to the species of host animal, type of parasite to be treated, severity of infection or infestation, and the body weight of the host. The amount of the composition producing the optimal effect is, of course, dependent on the particular active compound, species of host animal to be treated, and the type and degree of parasitism or prevalence. Moreover, the rate of release of the active ingredient can be freely adjusted by various techniques such as microencapsulation.

From field testing with the combination of pyraclofos and albendazole, the effective ranges of dosages for internal administration include pyraclofos from about 5 to about 50 mgs/kg animal body weight and albendazole from about 1.0 to about 10.0 mgs/kg animal body weight. Preferably, for administration to sheep pyraclofos is present in the combination at about 30 mgs/kg with albendazole at 3.8 mgs/kg. For cattle, the albendazole dosage may be increased to 10 mgs/kg. These dosages can be adjusted for the effect of other ingredients if necessary.

Oral preparations contain generally about 1 to 80 percent by weight of the active compounds (pyraclofos in combination with albendazole) and preferably about 10 to 30 percent by weight. Generally, however, the useful oral dose of the active compound is about 0.1 mg to 2,000 mg and preferably about 1 mg to 1200 mg per kilogram body weight of the animal and this dose is preferably administered in a single oral dose. One presently desirable dosage contains about 40 mg/kg of the active compound.

For administration in feed premixes, the feed may desirably contain about 1 to 80 percent, preferably 10 to 30 percent by weight of the active-compound(s), i.e., pyraclofos with albendazole. Feed supplements for direct feeding to animals contain about 1 to 50 percent by weight, preferably about 5 to 25 percent by weight, of the active compound(s). Such feed supplements are added to animal rations in amounts sufficient to insure concentrations of the active compound which are effective for the treatment or management of parasitism. The preferred concentration of the active compound is dependent on the above-mentioned factors and the species of compound but in order to insure an adequate antiparasitic effect, it is generally recommended that the compound be used in a concentration of about 1.0 to 50.0 percent, preferably about 10 to 30 percent.

Preparations for administration by routes other than oral contain generally about 1 to 80 percent by weight and preferably about 10 to 50 percent by weight of the active compound(s). For parenteral administration, the useful dosage is generally about 0.1 mg to 300 mg/dose and preferably about 1 mg to 30 mg/dose per kilogram body weight of the animal.

It is preferable that animals be treated with repeated dosages of the compositions of this invention to inhibit reinfection or recurrence of original infection. Specifically, the compositions of the present invention may be administered repeatedly over a comparatively short period, for example 1 to 5 days for pet animals.

The following examples illustrate various aspects of the invention, but are not intended to limit the scope of the invention.

### Example 1 - Preparation of Compositions

The term "active ingredient" as used below means pyraclofos unless otherwise indicated.

### A. Albendazole

The benzimidazole Albendazole used in the following examples is the commercially available Valbazen [SmithKline Beecham Animal Health, Lincoln, NE] containing 19 grams per liter of the compound in an aqueous suspension.

### B. Pyraclofos

Several solutions of pyraclofos may be prepared as follows: Pyraclofos (20.0% W/W), phenyl phosphonate CALX (4.0 % W/W), Synperonic NP13 (6.0% W/W) and Solven 100 (70.0% W/W) are mixed and stirred to provide a solution, which is then diluted with water to give an emulsion of 250 ppm concentration.

An alternative solution is made by dissolving the active ingredient (30.0% W/W) in corn oil (70.0% W/W) to give a homogeneous solution for oral administration.

A granule formulations is prepared by dissolving the active ingredient (10.0% W/W) and either wood resin (4.0% W/W) or Synperonic NP 13 (4.0% W/W) in methylene chloride and the solution is added to granular gypsum (20∼60 mesh; 86.0% W/W) in a mixer. After sufficient mixing, the solvent is removed, followed by drying to give granules.

An alternative granule formulation is prepared by the wet granulation method. The active ingredient (25.0% W/W) is mixed with calcium sulfate hemihydrate (75.0% W/W). The mixture is then dried with a tray or fluidized-bed dryer and filled into a suitable container.

A tablet formulation is prepared by adding to the active ingredient (50.0% W/W) an adequate amount of 10% starch paste [magnesium stearate (1.0% W/W), corn starch (5.0% W/W), sodium starch glycolate (2.0% W/W), sodium laurylsulfate (1.0% W/W) and microcrystalline cellulose (41.0% W/W) to prepare a wet mass for granulation. The mass is granulated and dried with a tray or fluidized-bed dryer. The dry granules are sieved and the remaining components are added. The mixture is compression-molded to give tablets.

As necessary, the core tablets are further coated with a film-forming material, such as hydroxypropylmethylcellulose, in an aqueous or non-aqueous solvent system. A plasticizer as well as a suitable color may be incorporated in the film-forming material solution.

An oral liquid formulation is prepared by dissolving the active ingredient (5.0% W/W) in a mixture of Polysorbate 85 (5.0% W/W), benzyl alcohol (3.0% W/W) and propylene glycol (30.0% W/W). The solution is adjusted to pH 6.0 ∼ 6.5 with phosphate buffer and diluted with water (57.0% W/W) to a specified final volume. The liquid is filled into a drinking water vessel for use.

An oral paste formulation is prepared by dispersing aluminum distearate (5.0% W/W) at 50 ∼ 60°C in a mixture of coconut oil (85.5% W/W) and Polysorbate 85 (3.0% W/W). The dispersion is cooled to ambient temperature with continued stirring, followed by dispersion of saccharin sodium (2.5% W/W) and active ingredient (4.0% W/W) to give a paste.

Several injectable formulations are prepared as follows: One injectable formulation is prepared by dissolving 20.0 % W/W of the active ingredient in a mixture of 36.0 % W/W ethanol and 10% W/W surfactant (Synperonic PE L44) and the solution is diluted with 34.0 % W/W propylene glycol to a specified final volume. This solution is sterilized by the established pharmaceutical procedure, for example by filtration through a bacterial filter or autoclaving and aseptically filled into unit-dose containers.

Another injectable formulation is prepared by dissolving 10% W/W of the active ingredient in a mixture of 10% W/W Polysorbate 80 and 50% W/W glycerol formal, followed by addition of 1.0 % W/W benzyl alcohol. The solution is diluted with 29% W/W water for inject ion to a specified final volume. This solution is sufficiently sterilized by the established pharmaceutical procedure and aseptically filled into unit-dose containers.

Another injectable formulation is prepared by mixing 10.0% W/W active ingredient, 0.5% W/W carboxymethylcellulose (CMC) and 89.5 % W/W physiological saline to give a uniform suspension. The resulting injection is suitable for intraperitoneal or intravenous administration.

Still a further injectable formulation is prepared by mixing 10.0% W/W active ingredient, 20.0% W/W Sorpol 1200 and 70.0 % W/W physiological saline to give a uniform suspension. The resulting injection is suitable for intraperitoneal or intravenous administration.

### C. Pyraclofos plus Albendazole

Three experiment combination products are prepared by homogenizing in vegetable oil, containing 1% Brij 58, 1% cetyl alcohol and 1% colloidal silica, 10 mg/kg pyraclofos and 3.8 mg/kg albendazole. The other formulations were similar except that they contained 20 mg/kg pyraclofos and 3.8 mg/kg albendazole and 30 mg/kg pyraclofos and 3.8 mg/kg albendazole, respectively.

### Example 2 - Combination Field Testing

The following test was performed to evaluate the anthelmintic efficacy of graded dozes of pyraclofos with and without the addition of albendazole when used to treat sheep infected with benzimidazole resistant strains of adult Haemonchus contortus. Ostertagia circumcincta and Trichostrongylus colubriformis.

Albendazole alone and the combination product are as described in Example 1 above. Pyraclofos was used in solution of vegetable oil at 100g/L.

Benzimidazole resistant (BZ-R) strains of H. contortus (H/C), O. circumcincta (O/C) and T. colubriformis (T/C) were obtained from animals in the field. Infective larvae were harvested from incubation within 10 days of infection.

Fifty six nine-month old merino weaners of both sexes were made worm-free by treatment orally with ivermectin (0.2 mg/kg) at least 2 weeks prior to the initiation of the trial and maintained in above ground mesh floored pens. All sheep are uniquely identified by a numbered plastic ear tag, and fed ad libitum on a diet of pelleted lucerne with vitamin-mineral supplement. Eight treatment groups of seven sheep were used as set out in Table 2. Trial schedule was as follows: all sheep were infected on days 1, 2 and 3. At each of these three consecutive days all sheep received the following doses of infective larvae:
- H/C: 3000
- O/C: 2000
- T/C: 3000
Infective larvae were administered by intra oesophageal tube, each species being present in approximately 100 mL of water. The fecal egg count on all sheep was performed on day 23. On the basis of ranked body weight within each gender, sheep were randomly assigned to eight groups of seven sheep on day 24.

Treatments were administered on day 25. Individual dose volumes were determined and each dose prepared and placed in calibrated syringe labelled with the ear tag number of the intended recipient. All treatments were administered by direct percutaneous intraruminal puncture.

Seven days after treatment individual fecal egg counts and group bulk larval culture were undertaken. Sheep were slaughtered for total worn counts (TWC) on day 36. The abomasum and small intestine were dissected out and TWC determined by standard techniques.

The results of treatment are illustrated in Table 2 below. In the Table, W stands for Wether, E represent Ewe, 1 represents Merino short wool lambs, 2 represents crossbred long wood lambs, H/C represents H. contortus, O/C represents O. circumcincta, and T/C represents T. colubriformis.

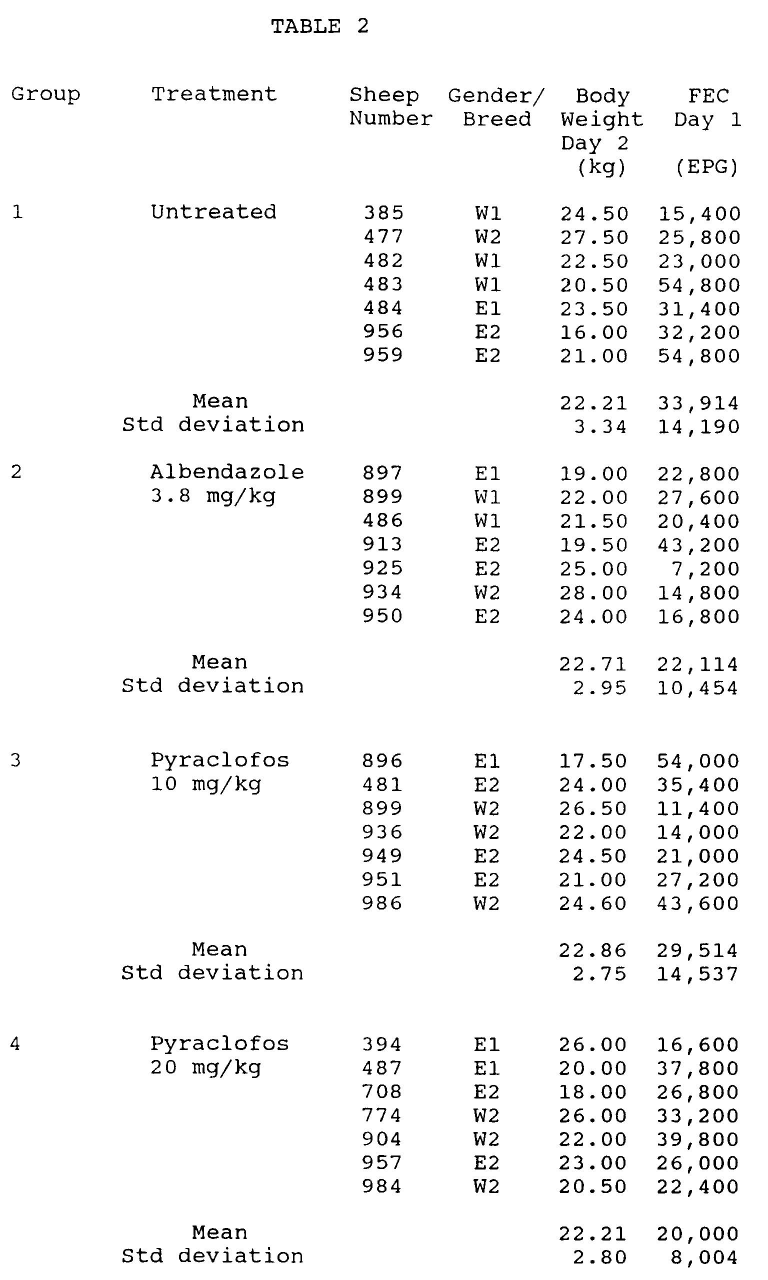

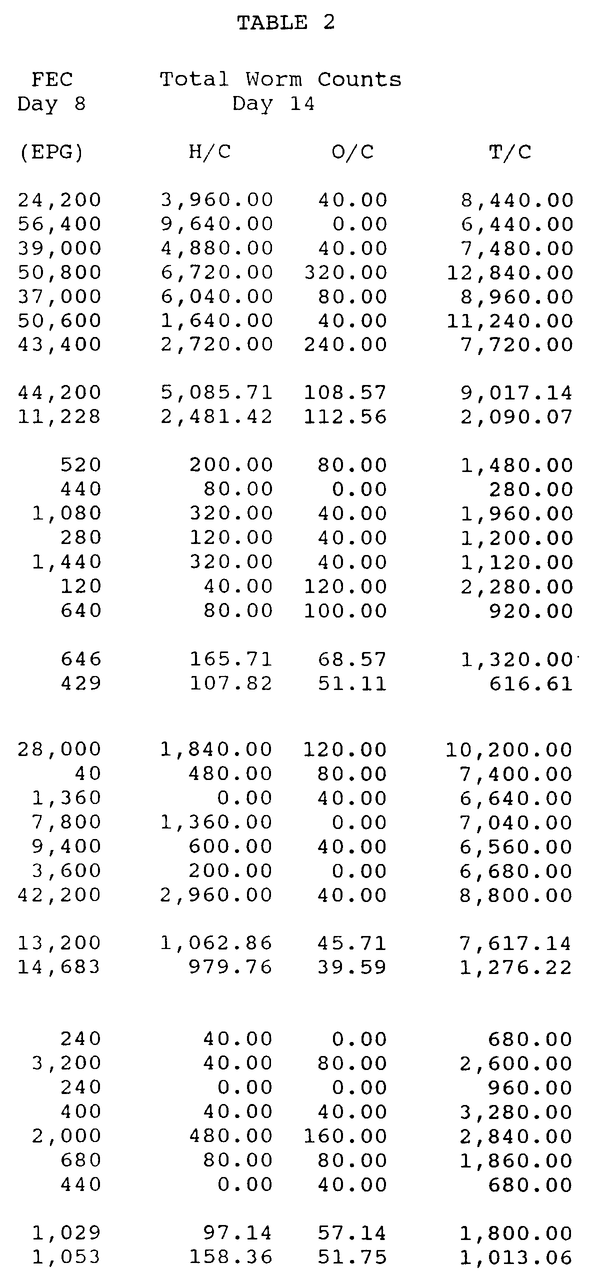

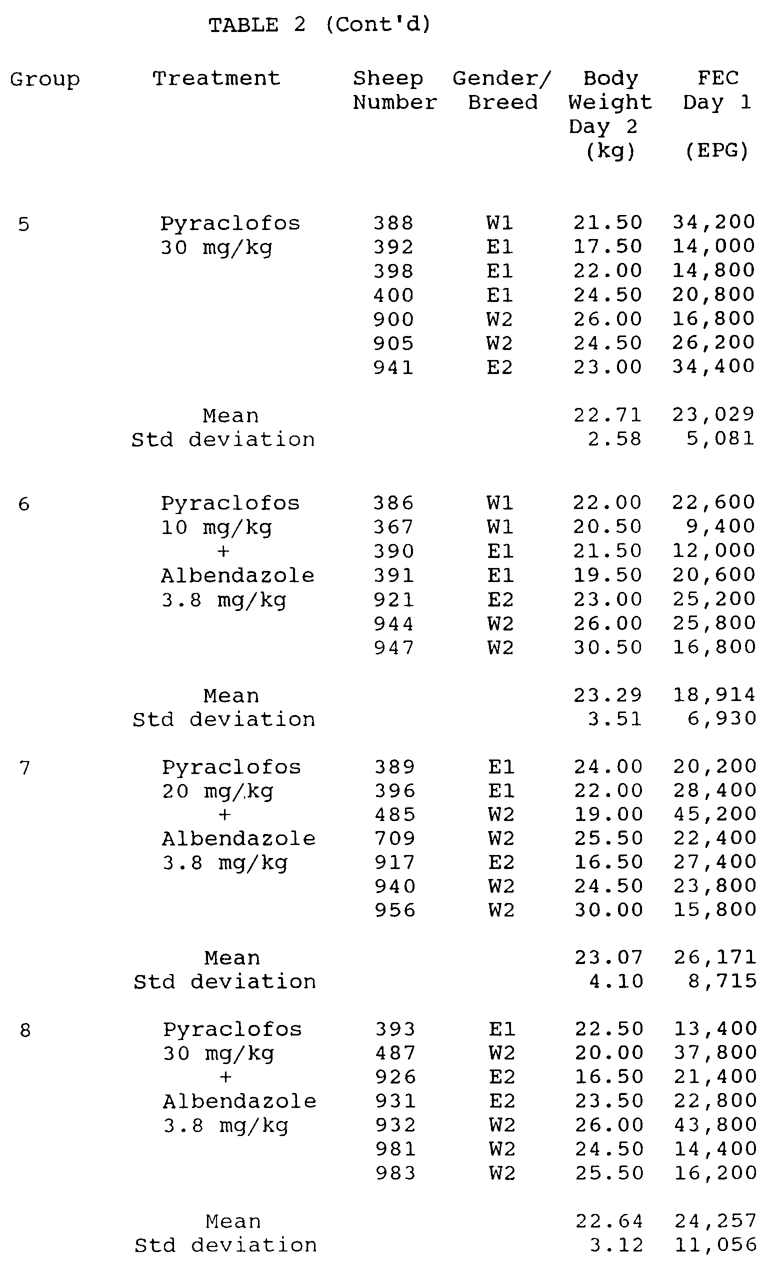

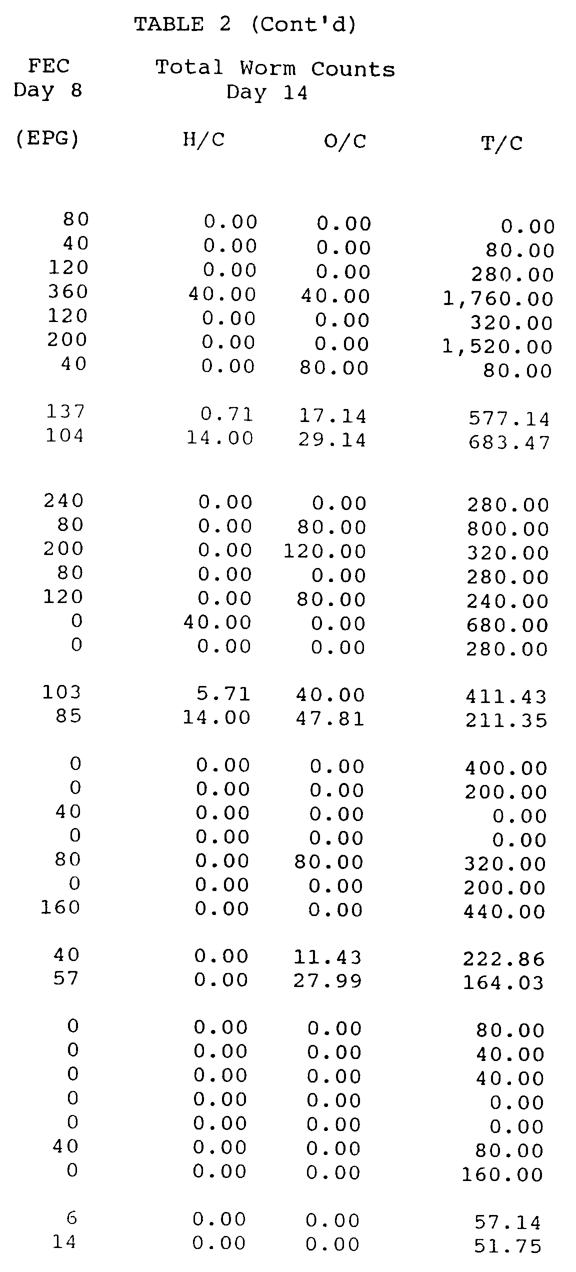

### Example 3 : Toxicity Study of Pyraclofos

The acute toxicity values (LD₅₀) in five-week old mice of ddY-SLC strain (male) are set forth below in Table 7.

**TABLE 7**

| Compound No. | LD₅₀(mg/kg) | Compound No. | LD₅₀(mg/kg) |
|---|---|---|---|
| 1 | >300 | 23 | 50∼300 |
| 2 | >300 | 24 | >300 |
| 3 | >300 | 25 | >300 |
| 4 | >300 | 26 | >300 |
| 5 | >300 | 27 | 50∼300 |
| 6 | >300 | 28 | >300 |
| 7 | >300 | 29 | ≒300 |
| 8 | >300 | 30 | >300 |
| 9 | >300 | 31 | >300 |
| 10 | >300 | 32 | >300 |
| 11 | >300 | 33 | >300 |
| 12 | ≒300 | 34 | >300 |
| 13 | >300 | 35 | ≒300 |
| 14 | >300 | 36 | >300 |
| 15 | >300 | 37 | >300 |
| 16 | >300 | 38 | >300 |
| 17 | >300 | 39 | |
| 18 | >300 | 40 | |
| 19 | >300 | 41 | 50∼300 |
| 20 | >300 | 42 | >300 |
| 21 | >300 | 43 | >300 |
| 22 | ≒300 | | |

Numerous modifications and variations of the present invention are included in the above-identified specification and are expected to be obvious to one of skill in the art. For example, use of other appropriate carriers, diluents and optional agents for use in the formulations may be part of this invention, as are obvious modes and intervals of administration. Such modifications and alterations to the compositions and processes of the present invention are believed to be encompassed in the scope of the claims appended hereto.

## Claims

1. An anti-endoparasitic composition comprising a combination of pyraclofos or a pyraclofos-related compound having the formula: 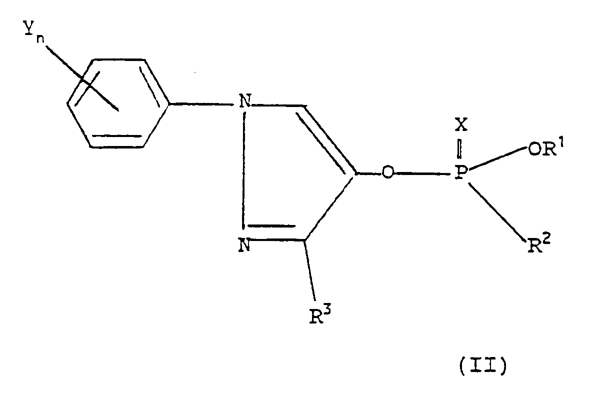 wherein R¹ is a lower alkyl group; R² is a lower alkoxy group or a lower alkylthio group; R³ is a hydrogen atom or a lower alkoxy carbonyl group; X is an oxygen atom or a sulfur atom; Y is a lower alkyl group, a lower alkoxy group, a lower alkylthio group, a halogen atom, a nitro group or a trifluoromethyl group; and n is an integer of 0, 1, 2 or 3; wherein the terms lower alkyl, lower alkoxy and lower alkylthio indicate groups containing 1 to 4 carbon atoms, lower alkoxycarbonyl means a group containing from 2 to 5 carbon atoms and halo means fluoro, chloro, bromo or iodo; and a benzimadazole carbamate selected from the group consisting of albendazole, fenbendazole, oxibendazole, oxfendazole, parbendazole, cambendazole, mebendazole, flubendazole, ricobendazole, and luxabendazole, or a prodrug derivative thereof selected from thiophanate, febantel or netobimin.

2. The composition according to claim 1 wherein said compound is pyraclofos and said benzimidazole carbamate is albendazole.

3. The composition according to claim 2 wherein said composition is an oral preparation containing from 1 to 80% of the active compounds pyraclofos and albendazole by weight.

4. The composition according to claim 3 wherein the amount of the active compounds pyraclofos and albendazole is 10 to 30% by weight.

## Patentansprüche

1. Antiendoparasitäre Zusammensetzung, umfassend eine Kombination von Pyraclofos, oder einer pyraclofosverwandten Verbindung, der Formel: 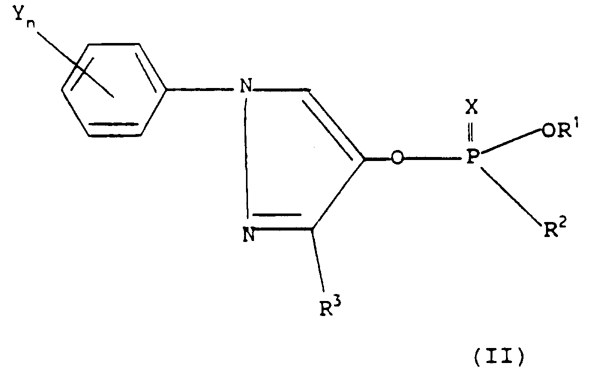 worin R¹ eine Niederalkylgruppe darstellt; R² eine Niederalkoxygruppe oder eine Niederalkylthiogruppe darstellt; R³ ein Wasserstoffatom oder eine Niederalkoxycarbonylgruppe darstellt; X ein Sauerstoffatom oder ein Schwefelatom darstellt; Y eine Niederalkylgruppe, eine Niederalkoxygruppe, eine Niederalkylthiogruppe, ein Halogenatom, eine Nitrogruppe oder eine Trifluormethylgruppe darstellt und n eine ganze Zahl von 0, 1, 2 oder 3 ist, wobei die Ausdrücke Niederalkyl, Niederalkoxy und Niederalkylthio Gruppen ausweisen, die 1 bis 4 Kohlenstoffatome enthalten, Niederalkoxycarbonyl eine 2 bis 5 Kohlenstoffatome enthaltende Gruppe bedeutet und Halogen Fluor, Chlor, Brom oder Jod bedeutet, und einen Benzimidazolcarbamat, ausgewählt aus der Gruppe, bestehend aus Albendazol, Fenbendazol, Oxibendazol, Oxfendazol, Parbendazol, Cambendazol, Mebendazol, Flubendazol, Ricobendazol und Luxabendazol oder einem Prodrugderivat davon, ausgewählt aus Thiophanat, Febantel oder Netobimin.

2. Zusammensetzung nach Anspruch 1, worin die Verbindung Pyraclofos darstellt und das Benzimidazolcarbamat Albendazol darstellt.

3. Zusammensetzung nach Anspruch 2, worin die Zusammensetzung eine orale Zubereitung ist, die 1 bis 80 Gew.-% der Wirkstoffe Pyraclofos und Albendazol enthält.

4. Zusammensetzung nach Anspruch 3, worin die Menge der Wirkstoffverbindungen Pyraclofos und Albendazol 10 bis 30 Gew.-% ist.

## Revendications

1. Composition anti-endoparasitaire, comprenant une association de pyraclofos ou d'un composé apparenté au pyraclofos répondant à la formule : 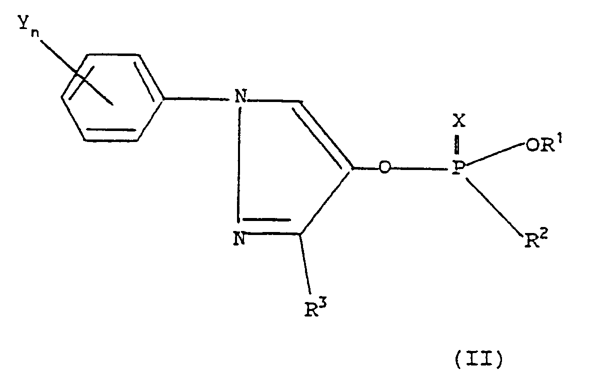 dans laquelle R¹ représente un groupe alkyle inférieur ; R² représente un groupe alkoxy inférieur ou un groupe alkylthio inférieur ; R³ représente un atome d'hydrogène ou un groupe alkoxycarbonyle inférieur ; X représente un atome d'oxygène ou un atome de soufre ; Y représente un groupe alkyle inférieur, un groupe alkoxy inférieur, un groupe alkylthio inférieur, un atome d'halogène, un groupe nitro ou un groupe trifluorométhyle ; et n est le nombre entier 0, 1, 2 ou 3 ; les expressions alkyle inférieur, alkoxy inférieur et alkylthio inférieur désignant des groupes contenant 1 à 4 atomes de carbone, l'expression alkoxycarbonyle inférieur désigne un groupe contenant 2 à 5 atomes de carbone et le terme halogéno désignant le groupe fluoro, chloro, bromo ou iodo ; et un benzimidazole-carbamate choisi dans le groupe consistant en albendazole, fenbendazole, oxibendazole, oxfendazole, parbendazole, cambendazole, mebendazole, flubendazole, ricobendazole et luxabendazole, ou un de ses dérivés promédicamenteux choisi entre le thiophanate, le fébantel et la nétobimine.

2. Composition suivant la revendication 1, dans laquelle le composé est le pyraclofos et le benzimidazole-carbamate est l'albendazole.

3. Composition suivant la revendication 2, qui est une préparation orale contenant 1 à 80 % des composés actifs consistant en pyraclofos et albendazole, en poids.

4. Composition suivant la revendication 3, dans laquelle la quantité des composés actifs consistant en pyraclofos et albendazole est comprise dans l'intervalle de 10 à 30 % en poids.
